# EUROPEAN PATENT APPLICATION

(11) **EP 4 501 131 A1**
(43) Date of publication of application: **05.02.2025**
(21) Application number: 22933669.8
(22) Date of filing: 20.12.2022
(51) Int. Cl.: A23L 5/00, A23L 29/10, A23L 29/20, A23L 29/30, A61K 8/06, A61K 8/34, A61K 8/60, A61K 8/73, A61K 9/107, A61K 47/10, A61K 47/36, A61K 47/40, B01F 23/41

(54) **HEAT-RESISTANT EMULSION COMPOSITION**

(30) Priority: 25.03.2022 JP 2022049691
(71) Applicant: House Wellness Foods Corporation, Hyogo 664-0011 (JP)
(72) Inventor: TOMOTAKE Muneaki, Itami-shi, Hyogo 664-0011 (JP); SHIMIZU Yusuke, Itami-shi, Hyogo 664-0011 (JP)
(74) Representative: Hoffmann Eitle
(86) International application number: PCT/JP2022/046861
(87) International publication number: WO 2023/181541

(57) **Abstract**

An object of the present invention is to provide an emulsified composition containing fat and oil, water, cyclodextrin, and a water-soluble gelling agent, which has high heat resistance and excellent emulsion stability without causing demulsification even when subjected to high-temperature conditions. Provided is an acidic emulsified composition containing water, fat and oil, cyclodextrin, a water-soluble gelling agent, and a water-soluble organic solvent.

## Description

### Technical Field

The present invention relates to an acidic emulsified composition containing water, fat and oil, cyclodextrin, a water-soluble gelling agent, and a water-soluble organic solvent.

### Background Art

Emulsified compositions containing fat and oil, water, cyclodextrin, and a water-soluble gelling agent nowadays are widely used in various fields of, for example, pharmaceuticals, cosmetics, and foods and drinks.

Patent Literature 1 discloses an emulsified composition containing water, an oily component, α-cyclodextrin, and one or more of agar, low-strength agar, a combination of galactomannan or glucomannan and xanthan gum, carrageenan, furcelleran, gellan gum, and native gellan gum as gel-forming components.

Patent Literature 2 discloses an emulsified composition characterized by containing fat and oil, water, cyclodextrin, and the like, and also discloses that gums such as xanthan gum, guar gum, gum arabic, CMC, carrageenan, and locust bean gum may be used as stabilizers.

Patent Literature 3 discloses an emulsified cosmetic obtained by mixing an aqueous raw material in which a water-soluble polymer compound such as pectin, tragacanth gum, gelatin, casein, sodium alginate, hydroxypropyl cellulose, hydroxyethyl cellulose, hydroxypropylmethyl cellulose, methyl cellulose, sodium polyacrylate, or carboxyvinyl polymer is dissolved with an oily raw material for a cosmetic, adding cyclodextrin thereto, and emulsifying the mixture.

Non Patent Literature 1 describes that emulsion stability increased when xanthan gum and tragacanth gum were each added as an emulsion stabilizer to an oil-drops-in-water type emulsion obtained by emulsifying a sample of soybean oil and an aqueous cyclodextrin solution at a volume ratio of 1:1.

### Citation List

### Patent Literature

Patent Literature 1: Japanese Patent Laid-Open No. 2016-204311
Patent Literature 2: Japanese Patent Laid-Open No. 10-262560
Patent Literature 3: Japanese Patent Publication No. 61-038166

### Non Patent Literature

Non Patent Literature 1: Nippon Shokuhin Kogyo Gakkaishi, Vol. 38, No. 1, 16-20 (1991)

### Summary of Invention

### Technical Problem

Conventional emulsified compositions containing fat and oil, water, cyclodextrin, and a water-soluble gelling agent have poor heat resistance in some cases and may fail to maintain the emulsified state and cause demulsification when subjected to high-temperature conditions in processes of product manufacturing, sterilization treatment, or the like. Therefore, it has been necessary to select a usage form that avoids high-temperature conditions, which has hindered the expansion of the applications of the emulsified compositions.

Therefore, an object of the present invention is to provide an emulsified composition containing fat and oil, water, cyclodextrin, and a water-soluble gelling agent, which has high heat resistance and excellent emulsion stability without causing demulsification even when subjected to high-temperature conditions.

### Solution to Problem

As a result of intensive studies to solve the problem, the present inventors have found that an acidic emulsified composition containing a water-soluble organic solvent together with fat and oil, water, cyclodextrin, and a water-soluble gelling agent has high heat resistance and excellent emulsion stability that allows the emulsified state to be maintained without causing demulsification even after being subjected to high-temperature conditions.

The present invention is based on these new findings, and the following inventions are included.
[1] An acidic emulsified composition containing water, fat and oil, cyclodextrin, a water-soluble gelling agent, and a water-soluble organic solvent.
[2] The composition according to [1], further including an acidic substance.
[3] A method for producing an acidic emulsified composition, including mixing water, fat and oil, cyclodextrin, a water-soluble gelling agent, and a water-soluble organic solvent to form an acidic emulsified composition.
[4] The method for producing an acidic emulsified composition according to [3], further including mixing an acidic substance.
[5] The method for producing an acidic emulsified composition according to [3] or [4], further including subjecting the acidic emulsified composition to a drying treatment.

The present specification encompasses the contents described in, for example, the specifications of Japanese Patent Application No. 2022-049691, filed on March 25, 2022, which is the basis of the priority of the present application.

All publications, patents, and patent applications cited herein are hereby incorporated by reference in their entirety.

### Advantageous Effects of Invention

According to the present invention, it is possible to provide an emulsified composition containing fat and oil, water, cyclodextrin, and a water-soluble gelling agent, which has high heat resistance and excellent emulsion stability without causing demulsification even when subjected to high-temperature conditions.

### Description of Embodiments

The present invention relates to an acidic emulsified composition containing water, fat and oil, cyclodextrin, a water-soluble gelling agent, and a water-soluble organic solvent (hereinafter, simply referred to as the "emulsified composition of the present invention" in some cases).

As the "fat and oil" in the present invention, fat and oil commonly used in an emulsified composition is available, and both polar and non-polar fats and oils may be used. Examples of fat and oil available in the present invention include, but are not limited to, plant-derived fat and oil (e.g., canola oil, refined rapeseed oil, soybean oil, corn oil, cottonseed oil, peanut oil, sesame oil, rice oil, rice bran oil, camellia oil, safflower oil, olive oil, linseed oil, Japanese basil oil, perilla oil, sunflower oil, palm oil, tea oil, coconut butter, avocado oil, *Aleurites moluccanus* seed oil, grapeseed oil, cocoa butter, coconut oil, wheatgerm oil, almond oil, evening primrose oil, castor oil, hazelnut oil, macadamia nut oil, rosehip oil, grape oil, cacao oil, jojoba oil, palm kernel oil, Japan wax, candelilla wax, and carnauba wax), synthetic ester oils such as paraffin, microcrystalline wax, ceresin, vaseline, ozokerite, isostearyl alcohol, caprylic alcohol, lauryl alcohol, stearyl alcohol, 2-octadecyl alcohol, myristyl alcohol, cetyl alcohol, phytosterol, cholesterol, stearic acid, isostearic acid, capric acid, lanolin acid, lauric acid, myristic acid, palmitic acid, behenic acid, linolic acid, linolenic acid, glyceryl monostearate, glyceryl monopalmitate, glyceryl monobehenate, glyceryl monomyristate, glyceryl monolaurate, glyceryl monolanolate, glyceryl monolinolate, glyceryl monolinolenate, glyceryl monooleate, glycerol triisostearate, isopropyl myristate, glycerol tri-2-heptylundecanoate, glycerol tri-2-ethylhexanoate, 2-heptylundecyl palmitate, di-2-heptylundecyl adipate, cetyl isooctanoate, trimethylolpropane-2-trimethylolheptylundecanoate, propane-2-ethylhexanoate, pentaerythritol-2-heptylundecanoate, pentaerythritol-2-ethylhexanoate, cholesterol isostearate, diethyl phthalate, and dibutyl phthalate, animal-derived fats and oils such as beef tallow, lard, lanolin, squalene, squalane, beeswax, and spermaceti, and silicone oil. In the present invention, the fat and oil, which can be appropriately selected depending on the utilization form in the intended application, is preferably animal-derived or plant-derived fat and oil with high safety and particularly preferably an edible vegetable oil that has been eaten and is highly safe. The fat and oil may be used alone or in combination with different fat and oil.

The emulsified composition of the present invention may contain fat and oil in any amount, for example, in an amount of 5 mass% or more, or 10 mass% or more. The upper limit thereof is not particularly limited, but may be, for example, 50 mass% or less, 40 mass% or less, 30 mass% or less, or 20 mass% or less. The range of the amount of fat and oil in the emulsified composition of the present invention can be expressed using two numerical values selected from each of the lower and upper numerical values. For example, the emulsified composition of the present invention may contain the fat and oil in an amount appropriately selected from the range of 5 mass% to 50 mass%, preferably 10 mass% to 30 mass%, and more preferably 10 mass% to 20 mass%. If the amount of fat and oil is less than 5 mass%, emulsification may be insufficient. On the other hand, if the amount of the fat and oil is more than 50 mass%, the emulsified composition may give strong oiliness such as stickiness or sliminess. The composition may not provide a desired feeling of use in the utilization form in the intended application.

In the present specification, the amount of each component is expressed as an amount of mass%, where the amount of the emulsified composition of the present invention is 100 mass%.

In the emulsified composition of the present invention, water may be contained in any amount capable of emulsifying fat and oil to form an oil-in-water emulsion together with other components. For example, the emulsified composition of the present invention may contain water in an amount of 15 mass% or more, 20 mass% or more, 30 mass% or more, 40 mass% or more, 45 mass% or more, 50 mass% or more, 60 mass% or more, or 70 mass% or more. The upper limit thereof is not particularly limited, but may be, for example, 90 mass% or less, or 85 mass% or less. The range of the amount of the water in the emulsified composition of the present invention can be expressed using two numerical values selected from each of the lower and upper numerical values. For example, the emulsified composition of the present invention may contain water in an amount appropriately selected from the range of 15 mass% to 90 mass%, preferably 45 mass% to 90 mass%, and more preferably 70 mass% to 85 mass%. The amount of water contained in the emulsified composition of the present invention is preferably more than that of fat and oil in volume ratio. For example, the content of fat and oil and water can be greater than 1:1 (fat and oil: water) in volume ratio, such as 1:2 or more, 1:3 or more, 1:4 or more, 1:5 or more, or 1:6 or more. The upper limit of the amount of water is not particularly limited, but can be 1:10 or less, preferably 1:8 or less.

"Cyclodextrin" means a cyclic non-reducing maltooligosaccharide having glucose as a constituent unit, and examples thereof include α-cyclodextrin having six glucose units, β-cyclodextrin having seven glucose units, and γ-cyclodextrin having eight glucose units. In the present invention, α-, β-, and γ-cyclodextrins, cyclodextrin derivatives, and any combination thereof can be used. Examples of the cyclodextrin derivatives include, but are not limited to, ethyl cyclodextrin, methyl cyclodextrin, hydroxyethyl cyclodextrin, hydroxypropyl cyclodextrin, methylamino cyclodextrin, amino cyclodextrin, carboxyethyl cyclodextrin, carboxymethyl cyclodextrin, sulfoxyethyl cyclodextrin, sulfoxyl cyclodextrin, acetyl cyclodextrin, branched cyclodextrin, cyclodextrin fatty acid ester, glucosyl cyclodextrin, and maltosyl cyclodextrin. Preferably, α-cyclodextrin is used. Since α-cyclodextrin has a high solubility in water, an emulsified composition with less roughness can be obtained.

The emulsified composition of the present invention may contain cyclodextrin in an amount capable of imparting emulsion stability to an oil-in-water emulsion and providing a desired feeling of use in the utilization form in the intended application. For example, the emulsified composition of the present invention may contain cyclodextrin in an amount of 1 mass% or more, 2 mass% or more, 3 mass% or more, 4 mass% or more, or 5 mass% or more. The upper limit thereof is not particularly limited, but may be, for example, 15 mass% or less, 10 mass% or less, 9 mass% or less, 8 mass% or less, 7 mass% or less, or 6 mass% or less. The range of the amount of the cyclodextrin in the emulsified composition of the present invention can be expressed using two numerical values selected from each of the lower and upper numerical values. For example, the emulsified composition of the present invention may contain the cyclodextrin in an amount appropriately selected from the range of 1 mass% to 15 mass%, for example, 2 mass% to 10 mass%, 3 mass% to 9 mass%, 4 mass% to 8 mass%, or 5 mass% to 7 mass%. If the amount of the cyclodextrin is less than 1 mass%, the emulsion stability of the composition may be insufficient. On the other hand, if the amount of the cyclodextrin is more than 15 mass%, the composition may have too high viscosity or give a strong creaky feeling. In either case, the composition may not provide a desired feeling of use in the utilization form in the intended application.

In the present invention, the "water-soluble gelling agent" generally means a substance that dissolves in water and imparts viscosity (sometimes also referred to as a thickener, a thickening stabilizer, or an adhesive, for example). Examples of the water-soluble gelling agent available in the present invention include, but are not limited to, xanthan gum, hydroxymethyl propyl cellulose, and methylcellulose, which can be appropriately selected depending on the utilization form in the intended application. Each of the water-soluble gelling agents may be used alone or in combination with different water-soluble gelling agents. More preferably, the water-soluble gelling agents are xanthan gum, hydroxymethyl propyl cellulose, and methylcellulose, and particularly preferably xanthan gum and hydroxymethyl propyl cellulose. These water-soluble gelling agents can impart high emulsion stability to the emulsified composition of the present invention, and in particular, can impart high heat resistance and excellent emulsion stability without causing demulsification even when subjected to high-temperature conditions.

The emulsified composition of the present invention may contain a water-soluble gelling agent in an amount capable of imparting emulsion stability to an oil-in-water emulsion and providing a desired feeling of use in the utilization form in the intended application. For example, the emulsified composition of the present invention may contain the water-soluble gelling agent in an amount of 0.1 mass% or more, 0.2 mass% or more, or 0.3 mass% or more. The upper limit thereof is not particularly limited, but may be, for example, 1 mass% or less, 0.8 mass% or less, 0.7 mass% or less, 0.6 mass% or less, or 0.5 mass% or less. The range of the amount of the water-soluble gelling agent in the emulsified composition of the present invention can be expressed using two numerical values selected from each of the lower and upper numerical values. For example, the emulsified composition of the present invention may contain the water-soluble gelling agent in an amount appropriately selected from the range of 0.1 mass% to 1 mass%, preferably 0.2 mass% to 0.8 mass%, and more preferably 0.2 mass% to 0.5 mass%. If the amount of the water-soluble gelling agent is less than 0.1 mass%, the composition may fail to have the desired emulsion stability and to maintain the emulsified state due to demulsification, particularly when subjected to high-temperature conditions. On the other hand, if the amount of the water-soluble gelling agent is more than 1 mass%, the composition may have too high viscosity. In either case, the composition may not provide a desired feeling of use in the utilization form in the intended application.

In the present invention, the term "water-soluble organic solvent" generally means an organic solvent that dissolves in water to form a homogeneous phase without forming a two-phase system (consisting of an organic phase and an aqueous phase) when mixed with water. Examples of the water-soluble organic solvent available in the present invention include alcohols, polyalcohols, glycol ethers, alkanediols, amines, amides, ketones, nitriles, nitrogen-containing solvents, and sulfur-containing solvents, which can be appropriately selected depending on the utilization form in the intended application.

Examples of the alcohols include, but are not particularly limited to, methanol, ethanol, propanol, isopropanol, butanol, isobutanol, 2-butanol, and tert-butanol.

Examples of the polyalcohols include, but are not particularly limited to, ethylene glycol, diethylene glycol, triethylene glycol, tetraethylene glycol, polyethylene glycol, propylene glycol, dipropylene glycol, tripropylene glycol, polypropylene glycol, butylene glycol, hexanediol, pentanediol, glycerin, hexanetriol, thiodiglycol, trimethylolpropane, xylitol, xylose, glucose, and galactose.

Examples of the glycol ethers include, but are not particularly limited to, ethylene glycol monomethyl ether, ethylene glycol monoethyl ether, ethylene glycol monobutyl ether, diethylene glycol monomethyl ether, diethylene glycol monoethyl ether, diethylene glycol monobutyl ether, triethylene glycol monobutyl ether, propylene glycol monopropyl ether, dipropylene glycol monomethyl ether, and tripropylene glycol monomethyl ether.

Examples of the alkanediols include, but are not particularly limited to, 1,2-butanediol, 1,3-propanediol, 1,3-butanediol, 1,4-butanediol, 1,2-pentanediol, 1,5-pentanediol, 2-methyl-2,4-pentanediol, 3-methyl-1,5-pentanediol, 1,6-hexanediol, 1,2-hexanediol, 1,8-octanediol, 1,2-octanediol, 1,9-nonanediol, 3-methyl-1,5-pentanediol, 2-methyl-1,3-propanediol, 2,2-dimethyl-1,3-propanediol, 2-butyl-2-ethyl-1,3-propanediol, 2,2-dibutyl-1,3-propanediol, 2-methyl-2,4-pentanediol, 2-methyl-1,8-octanediol, 1,4-cyclohexanediol, and 1,4-cyclohexanedimethanol.

Examples of the amines include, but are not particularly limited to, ethanolamine, diethanolamine, triethanolamine, N-methyldiethanolamine, N-ethyldiethanolamine, morpholine, N-ethylmorpholine, ethylenediamine, diethylenediamine, triethylenetetramine, tetraethylenepentamine, polyethyleneimine, pentamethyldiethylenetriamine, and tetramethylpropylenediamine.

Examples of the amides include, but are not particularly limited to, formamide, N,N-dimethylformamide, and N,N-dimethylacetamide.

Examples of the ketones include, but are not particularly limited to, acetone, methyl ethyl ketone, methyl isobutyl ketone, acetylacetone, diacetone alcohol, cyclopentanone, and cyclohexanone.

Examples of the nitriles include, but are not particularly limited to, acetonitrile and propionitrile.

Examples of the nitrogen-containing solvents include, but are not particularly limited to, 2-pyrrolidone, N-methyl-2-pyrrolidone, N-hydroxyethyl-2-pyrrolidone, cyclohexylpyrrolidone, and triethanolamine.

Examples of the sulfur-containing solvents include, but are not particularly limited to, thiodiethanol, thiodiglycerol, sulfolane, and dimethyl sulfoxide.

In the present invention, each of the water-soluble organic solvents may be used alone or in combination with different water-soluble organic solvents. More preferably, the water-soluble organic solvents are lower alcohols (methanol, ethanol, propyl alcohol, etc.), acetone, or acetonitrile. The water-soluble organic solvents can impart high emulsion stability to the emulsified composition of the present invention, and in particular, can impart high heat resistance and excellent emulsion stability without causing demulsification even when subjected to high-temperature conditions.

In the emulsified composition of the present invention, the water-soluble organic solvent may be contained in an amount capable of imparting emulsion stability to an oil-in-water emulsion and providing a desired feeling of use in the utilization form in the intended application. For example, the emulsified composition of the present invention may contain the water-soluble organic solvent in an amount of 0.02 mass% or more, 0.1 mass% or more, 0.5 mass% or more, 1 mass% or more, 2 mass% or more, 3 mass% or more, 4 mass% or more, or 5 mass% or more. The upper limit thereof is not particularly limited, but may be, for example, 15 mass% or less, 10 mass% or less, 9 mass% or less, 8 mass% or less, 7 mass% or less, or 6 mass% or less. The water-soluble organic solvent may be blended in the emulsified composition of the present invention as a raw material of the emulsified composition and/or may be blended in a form contained in an additive such as a perfume or a functional formulation. The range of the amount of the water-soluble organic solvent in the emulsified composition of the present invention can be expressed using two numerical values selected from each of the lower and upper limit numerical values. For example, the emulsified composition of the present invention may contain the water-soluble organic solvent in an amount appropriately selected from the range of 0.02 mass% to 15 mass%, for example, 0.1 mass% to 10 mass%, 0.5 mass% to 9 mass%, 1 mass% to 8 mass%, or 2 mass% to 7 mass%. If the amount of the water-soluble organic solvent is less than 0.02 mass%, the composition may fail to have the desired emulsion stability, to maintain the emulsified state due to demulsification, particularly when subjected to high-temperature conditions, and to provide a desired feeling of use in the utilization form in the intended application.

In the present invention, the term "acidic" means that the pH value of the emulsified composition of the present invention at 25°C is less than 7, preferably 6.5 or less, more preferably 6 or less, even more preferably 5.5 or less, and still more preferably 5 or less, for example, 4.5 or less, 4 or less, 3.5 or less, or 3 or less. The lower limit thereof is not particularly limited, but may be, for example, 1 or more, for example, 1.5 or more, 2 or more, or 2.5 or more. The range of the pH value in the emulsified composition of the present invention at 25°C can be expressed using two numerical values selected from each of the lower and upper limit numerical values. For example, the pH value of the emulsified composition of the present invention at 25°C may be appropriately selected from the range of 1 to 6.5, 1.5 to 6.5, 2 to 6.5, or 3 to 6.5. When the emulsified composition of the present invention is not acidic, i.e., when the pH value is 7 or more, the composition may fail to have the desired emulsion stability and to maintain the emulsified state due to demulsification, particularly when subjected to high-temperature conditions. On the other hand, when the pH is lower than 1, the acidity may become too strong. In either case, the composition may not provide a desired feeling of use in the utilization form in the intended application. Note that the pH value of the emulsified composition of the present invention is a value measured at 25°C according to JIS Z8802: 2011.

The emulsified composition of the present invention may be blended with an acidic substance so as to be "acidic". The "acidic substance" available in the present invention may be any substance that can make the emulsified composition of the present invention acidic as described above, and examples thereof include an organic acid and an inorganic acid and salts thereof, which can be appropriately selected depending on the utilization form in the intended application. More specifically, the acidic substance includes a substance generally used as a pH adjuster, and examples thereof include, but are not limited to, organic acids such as citric acid, ascorbic acid, gluconic acid, glycolic acid, malic acid, tartaric acid, succinic acid, lactic acid, acetic acid, formic acid, oxalic acid, malonic acid, glutaric acid, adipic acid, pimelic acid, phthalic acid, isophthalic acid, terephthalic acid, propionic acid, butyric acid, valeric acid, gluconic acid, glyceric acid, tartronic acid, salicylic acid, gallic acid, tropic acid, fumaric acid, maleic acid, glutamic acid, aspartic acid, hydroxyacrylic acid, and α-oxybutyric acid; inorganic acids such as hydrochloric acid, sulfuric acid, sulfurous acid, perchloric acid, carbonic acid, phosphoric acid, polyphosphate, hydrobromic acid, hydroiodic acid, hydrofluoric acid, and hydrochloric acid; and salts thereof. Examples of the "salts" include, but are not limited to, alkali metal salts such as sodium, potassium, and lithium, alkaline earth metal salts such as magnesium and calcium, ammonium salts, and substituted ammonium salts. Each of the acidic substances may be used alone or in combination with different acidic substances. Especially, citric acid, ascorbic acid, salts thereof are preferred from the viewpoint of safety and solubility in water.

In the emulsified composition of the present invention, the acidic substance can be contained in an amount capable of making the emulsified composition of the present invention "acidic" as described above. The blending amount of the acidic substance may vary depending on factors such as the type of the acidic substance to be used and the type and amount of other components blended in the emulsified composition of the present invention, but may be, for example, 0 mass% or more, more than 0 mass%, 1 mass% or more, 2 mass% or more, 3 mass% or more, 4 mass% or more, or 5 mass% or more. The upper limit thereof is not particularly limited, but may be, for example, 15 mass% or less, 10 mass% or less, 9 mass% or less, 8 mass% or less, or 7 mass% or less. The range of the amount of the acidic substance in the emulsified composition of the present invention can be expressed using two numerical values selected from each of the lower and upper limit numerical values. For example, the emulsified composition of the present invention may contain the acidic substance in an amount appropriately selected from the range of 0 mass% to 15 mass%, 1 mass% to 15 mass%, 2 mass% to 10 mass%, 3 mass% to 9 mass%, 4 mass% to 8 mass%, or 5 mass% to 7 mass%. The emulsified composition of the present invention contains an acidic substance, which allows the emulsified composition of the present invention to be "acidic" as described above, and can impart high emulsion stability to the emulsified composition of the present invention, and in particular, can impart high heat resistance and excellent emulsion stability without causing demulsification even when subjected to high-temperature conditions.

Since the emulsified composition of the present invention may have high emulsion stability by blending each of the components, the emulsified composition may be substantially free of a synthetic surfactant and/or a polyoxyethylene glycol, each of which is generally used as an emulsifier in a conventional emulsified composition, in addition to the above described components. Preferably, the emulsified composition of the present invention is substantially free of a synthetic surfactant and/or polyoxyethylene glycol. In the present invention, the phrase "substantially free of a synthetic surfactant and/or polyoxyethylene glycol" means that the emulsified composition of the present invention does not contain a synthetic surfactant and/or polyoxyethylene glycol in such a manner as to exhibit an emulsifying effect, which is not intended to mean that the emulsified composition does not contain a synthetic surfactant and/or a polyoxyethylene glycol at all.

The emulsified composition of the present invention has high emulsion stability, and in particular, the emulsified composition of the present invention has high heat resistance and excellent emulsion stability that allows the emulsified state to be maintained even after being subjected to high-temperature (e.g., about 100°C to 130°C) conditions without causing demulsification.

The emulsified composition of the present invention is not limited to any particular form and may be provided in a form of a semi-solid/semi-liquid (e.g., a gel or a sol) or a dried body (e.g., a powder or a flake) prepared by reducing the water content, in addition to a form having the above-described water content. Such a form of a semi-solid/semi-liquid (e.g., a gel or a sol) or a dried body (e.g., a powder or a flake) can be formed after obtaining an emulsified composition in a form having the above water content by subjecting the emulsified composition to the conventionally known drying method to reduce the water content of the emulsified composition. Examples of such a drying method include, but are not limited to, freeze drying, heat drying, air drying, spray drying, drum drying, hot air drying, and vacuum drying. The water content of the emulsified composition after being subjected to the drying method can be appropriately selected depending on the intended form, and may be, for example, less than 15 mass%, 10 mass% or less, 5 mass% or less, or 1 mass% or less. The lower limit of the water content of the emulsified composition after being subjected to the drying method is not particularly limited but preferably can be 0.1 mass% or more, 0.5 mass% or more, 0.6 mass% or more, 0.7 mass% or more, or 0.8 mass% or more. The range of the water content of the emulsified composition after being subjected to the drying method can be expressed using two numerical values selected from each of the lower and upper numerical values. For example, the water content of the emulsion composition may be 0.1 mass% to less than 15 mass%, 0.1 mass% to 10 mass%, 0.5 mass% to 5 mass%, 0.5 mass% to 1 mass%, 0.6 mass% to 1 mass%, 0.7 mass% to 1 mass%, or 0.8 mass% to 1 mass%. The emulsified composition after being subjected to the drying method may be used as it is or may be used after re-emulsification by adding an appropriate solvent such as water thereto/adding the composition to an appropriate solvent such as water at the time of use.

The emulsified composition of the present invention can be used and contained as a base in applications, but not limited to, for foods and beverages, cosmetics, topical drugs, pharmaceuticals (including quasi-drugs), chemical agents, chemical products, agrochemicals, toiletries, spray products, paints, industrial thin film materials, surface modifying materials, or the like, and can be provided in a utilization form (e.g., a form of a prescribed product or the like) in the intended application.

The emulsified composition of the present invention has high emulsion stability, and in particular, the emulsified composition of the present invention can maintain the emulsified state even after being subjected to high-temperature (e.g., about 100°C to 130°C) conditions. The high emulsion stability allows the utilization form of the emulsified composition of the present invention as a base in the intended application to be subjected to high-temperature conditions in processes of production, sterilization, storage, use, or the like. For example, the utilization form of the emulsified composition of the present invention as a base in the intended application can be subjected to a heat sterilization treatment, more specifically to a high-temperature sterilization treatment (e.g., retort sterilization treatment) at 100°C to 130°C, thus providing a highly safe product with low/no antiseptic content. For example, the emulsified composition of the present invention can be subjected to high-temperature conditions together with other raw material or can be immediately combined with other raw materials subjected to high-temperature conditions, which not only contributes to the efficiency of the production process and operation but also enables the production of utilization forms (e.g., prescribed products) that could not be achieved before due to the avoidance of high-temperature conditions.

In addition to the above-described components, for the emulsified composition of the present invention, if necessary, components usually used in the production of the utilization form in the intended application can further be appropriately blended according to the desired utilization form, as long as the effects of the present invention are not impaired. Examples of such components include, but are not limited to, excipients, disintegrators, lubricants, binders, diluents, buffers, suspending agents, thickeners, preservatives, antibacterial agents, antiseptics, antioxidants, ultraviolet absorbers, coloring agents, pigments, dyes, colorants, lubricants, plasticizers, solvents, solubilizers, isotonic agents, correctives, perfumes, sweeteners, taste components, acidulants, seasonings, humectants, vitamins, surfactants, chelating agents, and antibacterial agents.

The emulsified composition of the present invention can be produced by mixing and stirring the water, fat and oil, cyclodextrin, the water-soluble gelling agent, the water-soluble organic solvent, as well as the acidic substance and other components, if necessary, in the above-described amounts. All of the components may be mixed and stirred together, or each component may be added separately or sequentially in any combination (in any order), then mixed and stirred. The emulsified composition obtained by mixing and stirring may be subjected to a heat sterilization treatment. The emulsified composition obtained by mixing and stirring may be further subjected to a drying method, if necessary. The drying method can be performed by the conventionally known drying method, and the water content of the emulsified composition can be appropriately adjusted according to the intended form. The emulsified composition subjected to the drying method may be further subjected to crushing, grinding, or abrasion treatment, if necessary, and other components may be mixed and stirred in the above-described amounts, if necessary.

Hereinafter, the present invention will be described with reference to Examples, but the present invention is not limited to these Examples.

### Examples

### I. Preparation of emulsified composition

According to the composition in Tables 1 to 6 below, each component was added and mixed to prepare emulsified compositions of Examples 1 to 16 and Comparative Examples 1 to 4. Each component was mixed and stirred using a hand blender at 20,000 rpm for 10 minutes. Each obtained emulsified composition was evaluated for emulsion stability by the following method.

The amount of each component in the tables is expressed as an amount of mass%, where the amount of the obtained emulsified composition is 100 mass%.

### II. Evaluation method of emulsion stability

The obtained emulsified compositions (10 mL) were each put in a 100 mL beaker and heat-treated in a microwave oven at 100°C (product temperature) for 1 minute. After the heat treatment, the emulsified compositions were allowed to stand at room temperature for 30 minutes. Then, the demulsification state (or emulsion stability) of each of the emulsified compositions was visually evaluated according to the following criteria.

### <Evaluation criteria>

⊚: No change. Emulsification was maintained.
○: No demulsification occurred, but some oil floating or separated-out water was observed.
×: Separation of oil and water layers was observed. Demulsification occurred.

### III. Evaluation result of emulsion stability

The evaluation results of the emulsion stability in the respective emulsified compositions are also shown in Tables 1 to 6 below.

**[Table 1]**

| Effect of pH on emulsion stability | | | | |
|---|---|---|---|---|
| | Example 1 | Example 2 | Example 3 | Example 4 |
| Palm oil | 10 | 10 | 10 | 10 |
| α-cyclodextrin | 7 | 7 | 7 | 7 |
| Water | 77.5 | 77.77 | 77.798 | 77.8 |
| Ethanol | 5 | 5 | 5 | 5 |
| Citric acid | 0.3 | 0.03 | 0.003 | 0 |
| Xanthan gum | 0.2 | 0.2 | 0.2 | 0.2 |
| Subtotal (mass%) | 100 | 100 | 100.001 | 100 |
| pH(25°C) | 3 | 3.5 | 5 | 6.5 |
| Emulsion stability | ⊚ | ⊚ | ⊚ | ○ |

**[Table 2]**

| Effect of water-soluble organic solvent on emulsion stability | | | | | | | | | |
|---|---|---|---|---|---|---|---|---|---|
| | Example 1 | Example 5 | Example 6 | Example 7 | Example 8 | Example 9 | Comparative Example 1 | Example 4 | Comparative Example 2 |
| Palm oil | 10 | 10 | 10 | 10 | 10 | 10 | 10 | 10 | 10 |
| α-cyclodextrin | 7 | 7 | 7 | 7 | 7 | 7 | 7 | 7 | 7 |
| Water | 77.5 | 79.5 | 81.5 | 82 | 82.4 | 82.48 | 82.5 | 77.8 | 82.8 |
| Ethanol | 5 | 3 | 1 | 0.5 | 0.1 | 0.02 | 0 | 5 | 0 |
| Citric acid | 0.3 | 0.3 | 0.3 | 0.3 | 0.3 | 0.3 | 0.3 | 0 | 0 |
| Xanthan gum | 0.2 | 0.2 | 0.2 | 0.2 | 0.2 | 0.2 | 0.2 | 0.2 | 0.2 |
| Subtotal | 100 | 100 | 100 | 100 | 100 | 100 | 100 | 100 | 100 |
| pH(25°C) | 3 | 3 | 3 | 3 | 3 | 3 | 3 | 6.5 | 6.5 |
| Emulsion stability | ⊚ | ⊚ | ⊚ | ⊚ | ⊚ | ○ | × | ○ | × |

**[Table 3]**

| Effect of water-soluble organic solvent type on emulsion stability | | | | |
|---|---|---|---|---|
| | Example 1 | Example 10 | Example 11 | Example 12 |
| Palm oil | 10 | 10 | 10 | 10 |
| α-cyclodextrin | 7 | 7 | 7 | 7 |
| Water | 77.5 | 77.5 | 77.5 | 77.5 |
| Ethanol | 5 | 0 | 0 | 0 |
| Acetone | 0 | 5 | 0 | 0 |
| Acetonitrile | 0 | 0 | 5 | 0 |
| Methanol | 0 | 0 | 0 | 5 |
| Citric acid | 0.3 | 0.3 | 0.3 | 0.3 |
| Xanthan gum | 0.2 | 0.2 | 0.2 | 0.2 |
| Subtotal | 100 | 100 | 100 | 100 |
| pH(25°C) | 3 | 3 | 3 | 3 |
| Emulsion stability | ⊚ | ⊚ | ⊚ | ⊚ |

**[Table 4]**

| Effect of water-soluble gelling agent on emulsion stability | | |
|---|---|---|
| | Example 1 | Example 13 |
| Palm oil | 10 | 10 |
| α-cyclodextrin | 7 | 7 |
| Water | 77.5 | 77.6 |
| Ethanol | 5 | 5 |
| Citric acid | 0.3 | 0.3 |
| Xanthan gum | 0.2 | 0.1 |
| Subtotal | 100 | 100 |
| pH(25°C) | 3 | 3 |
| Emulsion stability | ⊚ | ⊚ |

**[Table 5]**

| Effect of water-soluble gelling agent type on emulsion stability | | | | | |
|---|---|---|---|---|---|
| | Example 1 | Example 14 | Example 15 | Comparative Example 3 | Comparative Example 4 |
| Palm oil | 10 | 10 | 10 | 10 | 10 |
| α-cyclodextrin | 7 | 7 | 7 | 7 | 7 |
| Water | 77.5 | 77.5 | 77.5 | 77.5 | 77.5 |
| Ethanol | 5 | 5 | 5 | 5 | 5 |
| Citric acid | 0.3 | 0.3 | 0.3 | 0.3 | 0.3 |
| Xanthan gum | 0.2 | 0 | 0 | 0 | 0 |
| Hydroxymethyl propyl cellulose | 0 | 0.2 | 0 | 0 | 0 |
| Methyl cellulose | 0 | 0 | 0.2 | 0 | 0 |
| Dextrin | 0 | 0 | 0 | 0.2 | 0 |
| Hydroxypropylated distarch phosphate | 0 | 0 | 0 | 0 | 0.2 |
| Subtotal | 100 | 100 | 100 | 100 | 100 |
| pH(25°C) | 3 | 3 | 3 | 3 | 3 |
| Emulsion stability | ⊚ | ⊚ | ○ | × | × |

**[Table 6]**

| Effect of acidic substance type on emulsion stability | | |
|---|---|---|
| | Example 1 | Example 16 |
| Palm oil | 10 | 10 |
| α-cyclodextrin | 7 | 7 |
| Water | 77.5 | 77.5 |
| Ethanol | 5 | 5 |
| Citric acid | 0.3 | 0 |
| Ascorbic acid | 0 | 0.3 |
| Xanthan gum | 0.2 | 0.2 |
| Subtotal | 100 | 100 |
| pH(25°C) | 3 | 3 |
| Emulsion stability | ⊚ | ⊚ |

The results confirmed that an acidic emulsified composition containing water, fat and oil, cyclodextrin, a water-soluble gelling agent, and a water-soluble organic solvent maintained the emulsified state without causing demulsification even after having being subjected to heat treatment under high-temperature conditions (Examples 1 to 16). In particular, better emulsion stability was confirmed when the acidic substance was blended to lower the pH value (Table 1). In addition, it was confirmed that the type of the acidic substance to be added is not particularly limited, and various acidic substances can be used (Table 6).

Comparative Examples 1 and 2 are examples in which the water-soluble organic solvent was not contained, and it was confirmed that the emulsified state was not maintained by heat treatment under high-temperature conditions, resulting in demulsification, and that the comparative examples did not provide emulsion stability, unlike the examples containing the water-soluble organic solvent (Table 2).

On the other hand, the type of the water-soluble organic solvent to be added was not particularly limited, and it was confirmed that the composition maintained emulsified state after having being subjected to a heat treatment under a high-temperature condition without causing demulsification in the case where any water-soluble organic solvent was used (Table 3).

Comparative Examples 3 and 4 are examples in which the dextrin and hydroxypropylated distarch phosphate was blended, and it was confirmed that the emulsified state was not maintained by heat treatment under high-temperature conditions, resulting in demulsification, and that the comparative examples did not provide emulsion stability, unlike the examples (Table 2).

However, the type of the water-soluble gelling agent to be added was not particularly limited, and it was confirmed that the composition maintained emulsified state after having being subjected to a heat treatment under a high-temperature condition without causing demulsification in the case where various water-soluble gelling agents were used (Tables 4 and 5).

The results revealed that an emulsified composition having high heat resistance and excellent emulsion stability without causing demulsification even when subjected to high-temperature conditions can be obtained by blending fat and oil, water, cyclodextrin and a water-soluble gelling agent in combination with a water-soluble organic solvent to prepare an acidic emulsified composition (if necessary, blending an acidic substance).

## Claims

1. An acidic emulsified composition comprising water, fat and oil, cyclodextrin, a water-soluble gelling agent, and a water-soluble organic solvent.

2. The composition according to claim 1, further comprising an acidic substance.

3. A method for producing an acidic emulsified composition, comprising mixing water, fat and oil, cyclodextrin, a water-soluble gelling agent, and a water-soluble organic solvent to form an acidic emulsified composition.

4. The method for producing an acidic emulsified composition according to claim 3, further comprising mixing an acidic substance.

5. The method for producing an acidic emulsified composition according to claim 3 or 4, further comprising subjecting the acidic emulsified composition to a drying treatment.
